(19) 

**Europäisches Patentamt**

**European Patent Office**

**Office européen des brevets**

(11) **EP 3 865 500 A1**

(12) **EUROPÄISCHE PATENTANMELDUNG**

(43) Veröffentlichungstag:
**18.08.2021 Patentblatt 2021/33**

(51) Int Cl.:
***C07H 3/02*** (2006.01)　　　***C07H 1/06*** (2006.01)
***C07H 1/00*** (2006.01)

(21) Anmeldenummer: **20156862.3**

(22) Anmeldetag: **12.02.2020**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Savanna Ingredients GmbH
50189 Elsdorf (DE)**

(72) Erfinder:
• **JUNKLEWITZ, Anna
42119 Wuppertal (DE)**

• **MOHR, Stephan
53879 Euskirchen (DE)**
• **BUTZ, Steffen
52372 Kreuzau (DE)**
• **KOCH, Timo
50189 Elsdorf (DE)**

(74) Vertreter: **Fabry, Bernd
IP2 Patentanwalts GmbH
Schlossstrasse 523
41238 Mönchengladbach (DE)**

(54) **ALLULOSE IN KRISTALLINER FORM**

(57)　　Vorgeschlagen wird eine kristalline Allulose mit einer definierten Partikelgrößenverteilung, ein Verfahren zu ihrer Herstellung sowie ihre Verwendung. Die neue Allulosequalität zeichnet sich dadurch aus, dass sie die Haltbarkeit damit konfektionierter Endprodukte sowie deren sensorischen und geschmacklichen Eigenschaften verbessert.

**EP 3 865 500 A1**

**Beschreibung**

## GEBIET DER ERFINDUNG

Die Erfindung befindet sich auf dem Gebiet der Lebensmitteltechnologie und betrifft eine neue kristalline Allulose, die durch eine spezielle Partikelgrößenverteilung charakterisiert ist, ein Verfahren zu ihrer Herstellung sowie ihre Verwendung.

## TECHNOLOGISCHER HINTERGRUND

Zu den Zuckerersatzstoffen werden die Süßstoffe und die Zuckeralkohole gerechnet. Süßstoffe können auf natürlichem oder synthetischem Weg hergestellt werden. Sie zeichnen sich dadurch aus, dass sie keinen oder nur einen vernachlässigbar geringfügigen Nährwert besitzen, was sie für Menschen, die Diät halten, besonders interessant macht.

Süßstoffe scheidet der Körper entweder ganz oder größtenteils unverändert aus. Sie finden sich in vielen Light- und Diätprodukten. Zuckeraustauschstoffe sind Kohlehydrate, die nur einen geringen Anstieg des Blutzucker- und Insulinspiegels verursachen. Ihre Süßkraft beträgt 40 bis 70 Prozent der Süßkraft normalen Haushaltszuckers. Der Anteil von Zuckerersatzstoffen in Lebensmitteln und Getränken hat in den letzten Jahren kontinuierlich zugenommen. Im Rahmen der vorliegenden Erfindung soll unter dem Begriff "niedrigkalorisch" ein Brennwert von nicht mehr als 10, vorzugsweise nicht mehr als 5 und insbesondere von 1 bis 2 kcal verstanden werden.

Die industriell bedeutsamsten Zuckerersatzstoffe sind Acesulfam K und Aspartam. Es handelt sich hierbei um synthetische Stoffe, die in einigen Studien als krebsgefährdend bezeichnet werden. Diese bisher nicht bestätigten Befunde stärken jedoch den allgemeinen Trend hin zu "natürlichen" Zuckerersatzstoffen.

In den letzten Jahren haben Stevioside breite Aufmerksamkeit erlangt, da die darin enthaltenen Einzelstoffe wie Rebaudiosid A eine bis zu 1000fach stärkere Süßkraft als Haushaltszucker besitzen und dabei nichtkalorisch sind. Nachteilig ist jedoch, dass Stevioside allgemein einen harten, metallischen Nachgeschmack aufweisen und sich daher bislang nicht als Zuckerersatzstoffe haben etablieren können.

Diese Lücke könnte geschlossen werden durch die so genannten "seltenen Zuckern" wie Tagatose, Cellobiose oder Allulose (die synonym auch als Psicose bezeichnet wird). Hierbei handelt es sich um Einfachzucker, die zwar nicht an die Süßkraft von Haushaltszucker herankommen, vom menschlichen Körper aber nicht verstoffwechselt werden. Sie gelten zudem als "natürlich", da sie in der Natur zu finden sind, wenn auch nur in geringen Mengen. Sie eignen sich daher insbesondere zum Teilaustausch in zuckerhaltigen Lebensmitteln.

## STAND DER TECHNIK

Verschiedene Verfahren zur Herstellung von Allulose sind aus dem Stand der Technik bekannt, wie beispielsweise aus den Dokumenten WO 2018/087261 A1 (Pfeifer & Langen GmbH & Co. KG) oder WO 2016/135358 A1 (Tate & Lyle Technology Limited).

WO 2018 20103 A1 (GIVAUDAN) beansprucht eine Sweetener-Mischung aus Stevia und weiteren Süßstoffen wie z.B. Psicose. Psicose steht dabei aber nicht im Vordergrund, sondern Mogroside.

WO 2014 186084 A1 (PEPSICO) beansprucht eine Getränkezusammensetzung, die als Süßstoffkomponente Erythrol, Psicose und Rebaudiosid M enthält.

## AUFGABE DER ERFINDUNG

Die Haltbarkeit von Lebensmitteln wird mit Hilfe der Wasseraktivität bzw. des so genannten $a_w$-Wertes beschrieben. Er stellt ein Maß für das "verfügbare" oder "aktive" Wasser im Gegensatz zur bloßen Angabe des Wassergehalts dar. Die Bedeutung dieser Größe ergibt sich daraus, dass für die Haltbarkeit von Lebensmitteln nicht nur der reine Wassergehalt von Bedeutung ist, sondern auch, in welchem Maße das Wasser durch das Substrat gebunden ist. Die Wasseraktivität beeinflusst das Wachstum von Mikroorganismen, den Ablauf chemischer Prozesse wie Fettoxidation und nichtenzymatische Bräunung, die Aktivität von Enzymen, und die physischen Eigenschaften des Lebensmittels. Die Wasseraktivität ist definiert als Verhältnis des Wasserdampfpartialdrucks in dem Lebensmittel (p) zum Sättigungsdampfdruck von reinem Wasser (po) bei einer bestimmten Temperatur:

$$a_w = p/P_0$$

Die Wasseraktivität ist gleichbedeutend mit der (relativen) Gleichgewichtsfeuchtigkeit (RGF), das heißt der relativen Luftfeuchtigkeit, bei der das Lebensmittel (wiederum bei der gegebenen Temperatur) mit der Umgebungsluft im Gleichgewicht steht, also weder Wasser verliert noch aufnimmt. Allerdings wird die relative Luftfeuchtigkeit meistens in der Hilfsmaßeinheit Prozent angegeben, so dass man die relative Gleichgewichtsfeuchtigkeit berechnet als:

$$RGF = a_w * 100$$

Gleichzeitig entspricht also die Wasseraktivität ($a_w$) 0-1 einer relativen Luftfeuchte von 0-100 %. Die Messung der Wasseraktivität erfolgt im einfachsten Fall, indem eine Probe des Lebensmittels in einen hermetisch verschlossenen Behälter gebracht und mit einem Hygrometer die Luftfeuchtigkeit gemessen wird, die sich in dem Behälter einstellt.

Eine Absenkung des Wasserwertes - gleichbedeutend mit der Verminderung des verfügbaren Wassers - stellt damit eine dauerhafte Herausforderung für die Lebensmittelindustrie dar, um die Haltbarkeit deren Produkte zu verbessern.

Die Aufgabe der vorliegenden Erfindung hat somit darin bestanden, einen nichtkalorischen Zuckerersatzstoff in einer neuen Form zur Verfügung zu stellen, der die Haltbarkeit von Nahrungsmitteln verlängert und die geschmacklichen und sensorischen Eigenschaften von Endzubereitungen, welche Zucker bzw. Zuckerersatzstoffe enthalten, verbessert.

## BESCHREIBUNG DER ERFINDUNG

Gegenstand der vorliegenden Erfindung ist eine neue kristalline Allulosequalität, die durch mindestens eine der folgenden Auswahlregeln, die die Partikelgrößenverteilung beschreiben, charakterisiert ist:

| Parameter | Dispergierdruck [kPa] | $X_{c(min)}$ | $X_{Fe(max)}$ | $X_{area}$ |
|---|---|---|---|---|
| Minimum d10 [μm] | 5 | > 70 | > 100 | >85 |
| Minimum d50 [μm] | 5 | > 140 | > 220 | > 185 |
| Minimum d90[μm] | 5 | > 230 | > 340 | > 265 |
| b/l d50 | 5 | 0,52-1,0 | 0,52-1,0 | 0,52-1,0 |
| Minimum d10 [μm] | 20 | > 50 | > 70 | > 65 |
| Minimum d50 [μm] | 20 | > 120 | > 200 | > 180 |
| Minimum d90[μm] | 20 | > 220 | > 330 | > 260 |
| b/l d50 | 20 | 0,53-1,0 | 0,55-1,0 | 0,55-1,0 |
| Minimum d10 [μm] | 460 | > 10 | >8 | > 10 |
| Minimum d50 [μm] | 460 | > 40 | > 65 | > 50 |
| Minimum d90[μm] | 460 | > 130 | > 170 | > 120 |
| b/l d50 | 460 | 0,60-1,0 | 0,65-1,0 | 0,65-1,0 |

Die besagte bevorzugte Korngrößenverteilung wird mittels dynamischer Bildanalyse (Camsizer X2 der Firma Retsch mit X-Jet Modul) ermittelt, wobei ein Dispergierdruck von 5, 20 oder 460 kPa angelegt wurde. Die Auswertung erfolgte in drei Größenmodellen, nämlich:

- $X_c$(min) Partikelbreite, welche aus der schmalsten aller gemessenen Sehnen $X_c$ bestimmt wird;

- $X_{Fe}$(max) Partikellänge, welche aus dem längsten aller gemessenen Feret-Durchmesser $X_{Fe}$ bestimmt wird.

- $X_{area}$ äquivalenter Partikeldurchmesser, welcher dem Durchmesser eines flächengleichen Kreises entspricht

Der Wert "b/l d50" gibt jeweils das Breiten/Längenverhältnis der Kristalle beim d50 wieder. Es ist besonders bevorzugt, wenn dieser Wert zwischen 0,6 und 1,0, insbesondere zwischen 0,7 und 0,9 und optimal zwischen 0,75 und 0,8 liegt.

Überraschenderweise wurde gefunden, dass Allulose als Süßungsmittel den Wasserwert von konfektionierten Produkten senken und damit deren Haltbarkeit deutlich verbessern kann, wenn diese eine definierte Partikelgrößenverteilung wie oben beschrieben aufweist. Dies ist insbesondere deshalb überraschend und nicht zu erwarten gewesen, da ein Zusammenhang zwischen Durchmesser und Form von Kristallpartikeln und Wasserwert bisher nicht bekannt war. Gleichzeitig ist diese neue kristalline Allulosequalität in der Lage, die sensorischen und geschmacklichen Eigenschaften von oralen Zubereitungen in Summe und von Lebensmitteln im Besonderen in vielfacher Weise positiv zu beeinflussen, wie zum Beispiel:

- Verbesserung des Mundgefühls,

- Intensivierung und Stabilisierung der Farbe, z.B. in Orangenlimonade;

- Verstärkung der Geschmacksintensität, z.B. intensivere Fruchtigkeit der Tomate in Ketchup oder der Umaminote in Snackartiklen;

- Stabilisierung von Lebensmittelemulsionen, oder

- schnellere Löslichkeit von Instantprodukten

um nur einige zu nennen.

## ALLULOSE IN KRISTALLINER FORM

Allulose (Psicose) ist ein kalorienarmer Zucker mit einem ähnlichen süßen Geschmack von Zucker. Allulose ist einer von vielen verschiedenen Zuckern, die in der Natur in sehr geringen Mengen vorkommen. Allulose wurde erstmals in Weizen identifiziert und seitdem in bestimmten Früchten wie Jackfrucht, Feigen und Rosinen gefunden. Allulose ist natürlich in kleinen Mengen in einer Vielzahl von süßen Lebensmitteln wie Karamellsauce, Ahornsirup und braunem Zucker enthalten. Allulose wird vom Körper absorbiert, aber nicht metabolisiert, sodass sie nahezu kalorienfrei ist.

Die Herstellung von Allulose bzw. Psicose aus Fructose ist seit 1995 bekannt, wie die Auswahl der folgenden Schriften zeigt:

- H. Itoh et al, Journal of Fermentation Bioengeneering, 80(1), 1995, S. 101-103 veröffentlicht die Herstellung von D-Psicose aus D-Fructose durch immobilisierte D-Tagatose 3-Epimerase.

- N. Wagner et al in Organic Process Research Development 2012, 16, S. 323-330 bezieht sich auf praktische Aspekte des integrierten Betriebs von Biotransformation und simulierter Bewegungsbetttrennung (SMB) für die feinchemische Synthese. D-Psicose wird unter Verwendung der D-Tagatose Epimerase-katalysierten Epimerisierung aus D-Fructose hergestellt.

- N. Wagner et al, in Chemical Engineering Science 137 (2015) S. 423-435 bezieht sich auf die modellbasierte Kostenoptimierung eines integrierten Prozesses zur enzymatischen Herstellung von Psicose bei erhöhten Temperaturen.

- N. Wagner et al, in Angewandte Chemie Int. Ed. Engl. 2015, 54(14), S. 4182-6 offenbart eine trennungsintegrierte Kaskadenreaktion zur Überwindung thermodynamischer Einschränkungen bei der Seltenzucker-Synthese.

- Bosshart et al, in Biotechnology Bioengineering 2016, 113(2), S. 349-58 bezieht sich auf die Produktion der seltenen Zucker D-Psicose und L-Tagatose durch zwei künstlich hergestellte D-Tagatose-Epimerasen.

- N. Wagner, et al., in Journal of Chromatography A 2015, 1398, S. 47-56 offenbart ein Verfahren zur wirtschaftlichen Herstellung von d-Psicose durch simulierte Wanderbettchromatographie.

- Gegenstand der WO 2018 087261 A1 (PFEIFER&LANGEN) ist ein Verfahren zur Synthese eines Einfachzuckers, vorzugsweise von D-Allulose aus einem Edukt, vorzugsweise von D-Fructose unter heterogener oder homogener Katalyse, die eine chemische und/oder enzymatische Katalyse beinhaltet, wobei die Synthese in mindestens zwei in Reihe geschalteten Reaktoren durchgeführt wird und das aus dem ersten Reaktor austretende Reaktionsprodukt vor dem Eintritt in den zweiten Reaktor einer chromatographischen Trennung unterzogen wird. Vorzugsweise wird die chromatographische Trennung in ein simuliertes Wanderbett integriert.

In der Regel wird Allulose in Form eines Sirups kommerzialisiert, der nach einem der oben genannten Verfahren erhältlich ist. In einer bevorzugten Ausführungsform umfasst oder besteht der flüssige Allulose-Sirup aus folgenden Komponenten, bezogen auf die im Sirup enthaltene Trockenmasse:

(i) etwa 90 bis etwa 95 Gew.-% Allulose,

(ii) etwa 5 bis etwa 10 Gew.-% Fructose,

(iii) 0 bis etwa 5 Gew.-% weitere Kohlenhydrate, verschieden von Allulose und Fructose, mit der Maßgabe, dass sich die Mengenangaben zu 100 Gew.-% ergänzen. Der guten Ordnung halber sei darauf hingewiesen, dass Ausführungsformen, die sich zu mehr oder zu weniger als 100 Gew.-% ergeben, weder von der Erfindung eingeschlossen noch von den Ansprüchen umfasst werden. Der Fachmann ist in jedem Fall in der Lage, nach Maßgabe von Beschreibung und Beispielen solche Zusammensetzungen aufzufinden, die die technische Lehre erfüllen, ohne dazu erfinderisch tätig werden zu müssen.

Die wässrigen Zubereitungen werden anschließend durch Wasserentzug aufkonzentriert. Der Verdampfungsprozess findet typisch entweder in einem oder in zwei bis drei in Reihe geschalteten Verdampfern statt. Bei den Bauteilen kann es sich Fallfilm- oder Plattenverdampfer handeln, die direkt oder indirekt mit Prozesswärme betrieben werden, wobei die Brüden In den Verdampfern oder Abscheidern über Kopf abdestilliert werden, während man die Sumpfprodukte - also die jeweiligen Konzentrate - von einer Stufe in die nächste leitet. Dabei wird die Trockensubstanz in der Regel auf etwa 55 bis etwa 90 Gew.-% angehoben. Ausgehend von einer Allulose-Lösung mit einem Trockensubstanzgehalt von typisch etwa 10 bis 25 Gew.-% oder alternativ etwa 50 bis etwa 70 Gew.-% wird durch Verdampfung dann ein Konzentrat mit einer typischen Trockensubstanz von etwa 80 bis etwa 90 Gew.-% erhalten.

Die Verdampfung wird in der Regel bei einer Temperatur von etwa 50 bis etwa 75 °C durchgeführt. Wird der Verfahrensschritt nicht einstufig, sondern in zwei oder drei Stufen durchgeführt, hat es sich aus energetischen Gründen als vorteilhaft erwiesen, bei der Temperaturführung den Bereich zwischen 59 und 71 °C auszulassen, also in den Verdampfern in Temperaturbereichen darüber und darunter zu arbeiten. Falls erforderlich können die Verdampferkonzentrate mit einem Entfärbungsmittel, beispielsweise Aktivkohle, behandelt werden.

## KRISTALLISATION

Ein weiterer Gegenstand der vorliegenden Erfindung betrifft ein Verfahren zur Herstellung von kristalliner Allulose wie vorstehend beschrieben, umfassend die folgenden Schritte:

(a) Bereitstellen einer wässrigen Allulose-Zubereitung, vorzugsweise einem Verdampferkonzentrat ("Allulose-Sirup");

(b) Aufkonzentrieren der Zubereitung aus Schritt (a) bis die Sättigungskonzentration überschritten wird;

(c) Versetzen der übersättigten Zubereitung aus Schritt (c) mit Allulose-Impfkristallen; und

(d) Abkühlen der Zubereitung aus Schritt (c) mit einer Geschwindigkeit von 0,1 bis etwa 1 K/h bis ein Kristallgehalt von etwa 30 bis etwa 50 Gew.-% erreicht wird, sowie

(e) Abtrennen und gegebenenfalls Trocknen der Kristalle.

Die genannten Verdampferkonzentrate weisen in der Regel einen Feststoffgehalt von 55 bis 90 Gew.-% und vorzugsweise von etwa 75 bis etwa 85 Gew.-% auf. Sie werden vorzugsweise im übersättigten Bereich, nahe dem Sättigungspunkt, mit Impfkristallen versetzt, wobei etwa 1 bis etwa 3 und typisch 1,5 bis 2 Gew.-% Allulosekristalle - bezogen auf die Konzentrate - zum Einsatz kommen. Die Übersättigung wird anschließend durch Verdampfung, vorzugsweise aber langsames Abkühlen (0,1-1K/h) aufrechterhalten bis sich ein Kristallgehalt von etwa 30 bis etwa 50 Gew.-% einstellt. Die Kristalle werden dann abgetrennt und gegebenenfalls noch getrocknet.

## GEWERBLICHE ANWENDBARKEIT

Ein weiterer Gegenstand der Erfindung betrifft die Verwendung von kristalliner Allulose wie oben beschrieben zur Herstellung von oralen Zubereitungen. Diese können ausgewählt sein aus der Gruppe, die gebildet wird von Nahrungsmitteln, Mund- und Zahnpflegemitteln sowie pharmazeutischen Zubereitungen. Beispiele für geeignete Nahrungsmittel umfassen:

- Getränke,

- Backwaren,

- Getreideprodukte,

- Knabberartikel,

- Süßwaren,

- Milchprodukte,

- Fruchtzubereitungen,

- Würzmittel,

- Gemüsezubereitungen,

- Fleischwaren oder

- Gewürze und Gewürzmischungen.

Die oralen Zubereitungen können die kristalline Allulose dabei in Mengen von 0,1 bis etwa 50 Gew.-%, vorzugsweise etwa 1 bis etwa 15 und insbesondere etwa 2 bis etwa 10 Gew.-% - bezogen auf die Zubereitungen - enthalten.

**BEISPIELE**

**BEISPIEL 1**

*Kristallisation von Allulose-Lösungen*

1.000 Liter Allulose-Verdampferkonzentrat mit einem Trockensubstanzgehalt von 70 Gew.-% wurde mit 1 Gew.-% Aktivkohle versetzt und über 3 Stunden gerührt. Anschließend wurde die entfärbte Lösung filtriert und aufkonzentriert, bis eine übersättigte Lösung erhalten wurde. Diese wurde in einen Kristallisator überführt und mit 1,5 Gew.-% Allulosekristallen geimpft. Danach wurde das Filtrat mit einer Geschwindigkeit von 1 K/h abgekühlt, bis sich die ersten Kristalle abschieden. Die Kristallisationsgeschwindigkeit wurde auf 0,5 K/h reduziert bis eine Kristallmenge von etwa 45 Gew.-% bezogen auf die Trockenmasse der übersättigten Lösung erreicht war. Anschließend wurden die Kristalle abgetrennt und getrocknet; die Mutterlauge wurde in den Prozess zurückgeführt. Es wurde eine kristalline Allulose erhalten, die die folgende Partikelgrößenverteilung aufwies:

| Parameter | Dispergierdruck [kPa] | $X_{c(min)}$ | $X_{Fe(max)}$ | $X_{area}$ |
|---|---|---|---|---|
| Minimum d10 [μm] | 5 | 72 | 105 | 90 |
| Minimum d50 [μm] | 5 | 150 | 230 | 190 |
| Minimum d90[μm] | 5 | 235 | 350 | 270 |
| Minimum d10 [μm] | 20 | 55 | 75 | 75 |
| Minimum d50 [μm] | 20 | 125 | 220 | 195 |
| Minimum d90[μm] | 20 | 225 | 335 | 280 |
| Minimum d10 [μm] | 460 | 12 | 10 | 12 |
| Minimum d50 [μm] | 460 | 45 | 70 | 55 |
| Minimum d90[μm] | 460 | 135 | 175 | 135 |

**VERGLEICHSBEISPIEL V1**

*Kristallisation von Allulose-Lösungen*

1.000 Liter Allulose-Verdampferkonzentrat mit einem Trockensubstanzgehalt von 70Gew.-% wurde mit 1 Gew.-% Aktivkohle versetzt und über 3 Stunden gerührt. Anschließend wurde die entfärbte Lösung filtriert und aufkonzentriert, bis eine übersättigte Lösung erhalten wurde. Diese wurde in einen Kristallisator überführt. Danach wurde das Filtrat mit einer Geschwindigkeit von 5 K/h abgekühlt, bis sich die ersten Kristalle abschieden. Die Kristallisationsgeschwindigkeit wurde auf 1 K/h reduziert bis eine Kristallmenge von etwa 45 Gew.-% bezogen auf die Trockenmasse der übersättigten Lösung erreicht war. Anschließend wurden die Kristalle abgetrennt und getrocknet; die Mutterlauge wurde in den Prozess zurückgeführt. Es wurde eine kristalline Allulose erhalten, die die folgende Partikelgrößenverteilung aufwies:

| Parameter | Dispergierdruck [kPa] | $X_{c(min)}$ | $X_{Fe(max)}$ | $X_{area}$ |
|---|---|---|---|---|
| Minimum d10 [$\mu$m] | 5 | 60 | 90 | 75 |
| Minimum d50 [$\mu$m] | 5 | 130 | 210 | 175 |
| Minimum d90 [$\mu$m] | 5 | 215 | 320 | 255 |
| Minimum d10 [$\mu$m] | 20 | 40 | 60 | 50 |
| Minimum d50 [$\mu$m] | 20 | 110 | 185 | 170 |
| Minimum d90 [$\mu$m] | 20 | 210 | 315 | 250 |
| Minimum d10 [$\mu$m] | 460 | 2 | 1 | 2 |
| Minimum d50 [$\mu$m] | 460 | 30 | 55 | 40 |
| Minimum d90 [$\mu$m] | 460 | 110 | 150 | 110 |

**BEISPIEL 2, VERGLEICHBEISPIELE V2 und V3**

*Bestimmung des Wasserwertes*

Es wurden gleichartige Backwaren (Brownies) unter Verwendung von Saccharose (V2), erfindungsgemäßer kristalliner Allulose nach Beispiel 1 sowie kristalliner nach Vergleichsbeispiel **V1** hergestellt. Jeweils Proben von 20 g der drei Produkte wurden Behälter gegeben, hermetisch verschlossen und dann über 3 h bei 20 °C gelagert. Anschließend wurde mit einem Hygrometer die Luftfeuchtigkeit bestimmt, die sich im Gasraum über den Proben eingestellt hatte. Die Zusammensetzung der Produkte sowie die Luftfeuchtigkeitswerte sind in der nachfolgenden Tabelle A wiedergegeben.

**Tabelle A**

Zusammensetzung und Luftfeuchtigkeit

| Zusammensetzung | 2 | V2 | V3 |
|---|---|---|---|
| Vollei | 240,0 g | 240,0 g | 240,0 g |
| Salz | 2,0 g | 2,0 g | 2,0 g |
| Allulose gemäß Beispiel 1 | 260,0 g | - | - |
| Allulose gemäß Beispiel V1 | - | 260,0 g | - |
| Saccharose | - | - | 260,0 g |
| Butter | 230,0 g | 230,0 g | 230,0 g |
| Mehl Typ 405 | 70,0 g | 70,0 g | 70,0 g |
| Kakaopulver | 110,0 g | 110,0 g | 110,0 g |
| Aroma (Vanille) | 5,0 g | 5,0 g | 5,0 g |
| *Luftfeuchtigkeit [%]* | | | |
| - nach 6 h | 72 | 76 | 80 |

(fortgesetzt)

| *Luftfeuchtigkeit [%]* | | | |
|---|---|---|---|
| - nach 48 h | 71 | 75 | 75 |

Die Beispiele und Vergleichsbeispiele zeigen, dass mit der neuen Allulosequalität der Wasserwert in Lebensmitteln gesenkt und damit deren Haltbarkeit verbessert werden kann.

**Patentansprüche**

1. Allulose in kristalliner Form, **dadurch gekennzeichnet, dass** sie eine Partikelgrößenverteilung bestimmt nach der $X_{c(min)}$-Methode aufweist, für die eine, zwei oder alle drei der folgenden Auswahlregeln gelten:

| Dispergierdruck [kPa] | Minimum d10 [$\mu$m] | Minimum d50 [$\mu$m] | Minimum d90 [$\mu$m] |
|---|---|---|---|
| 5 | > 70 | > 140 | > 230 |
| 20 | > 50 | > 120 | > 220 |
| 460 | > 10 | > 40 | > 130 |

2. Allulose nach Anspruch 1, **dadurch gekennzeichnet, dass** diese einen b/l d50-Wert aufweist, für den gilt:

- Dispergierdruck 5 kPa : 0,52 bis 1,0 und/oder
- Dispergierdruck 20 kPa : 0,53 bis 1,0 und/oder
- Dispergierdruck 460 kPa : 0,60 bis 1,0.

3. Allulose in kristalliner Form, **dadurch gekennzeichnet, dass** sie eine Partikelgrößenverteilung bestimmt nach der $X_{Fe(max)}$-Methode aufweist, für die eine, zwei oder alle drei der folgenden Auswahlregeln gelten:

| Dispergierdruck [kPa] | Minimum d10 [$\mu$m] | Minimum d50 [$\mu$m] | Minimum d90 [$\mu$m] |
|---|---|---|---|
| 5 | > 100 | > 220 | > 340 |
| 20 | > 70 | > 200 | > 330 |
| 460 | > 8 | > 65 | > 170 |

4. Allulose nach Anspruch 3, **dadurch gekennzeichnet, dass** diese einen b/l d50-Wert aufweist, für den gilt:

- Dispergierdruck 5 kPa : 0,52 bis 1,0 und/oder
- Dispergierdruck 20 kPa : 0,55 bis 1,0 und/oder
- Dispergierdruck 460 kPa : 0,65 bis 1,0.

5. Allulose in kristalliner Form, **dadurch gekennzeichnet, dass** sie eine Partikelgrößenverteilung bestimmt nach der $X_{area}$-Methode aufweist, für die eine, zwei oder alle drei der folgenden Auswahlregeln gelten:

| Dispergierdruck [kPa] | Minimum d10 [$\mu$m] | Minimum d50 [$\mu$m] | Minimum d90 [$\mu$m] |
|---|---|---|---|
| 5 | > 85 | > 185 | > 265 |
| 20 | > 65 | > 180 | > 260 |

(fortgesetzt)

| Dispergierdruck [kPa] | Minimum d10 [$\mu$m] | Minimum d50 [$\mu$m] | Minimum d90 [$\mu$m] |
|---|---|---|---|
| 460 | > 10 | > 50 | > 120 |

6. Allulose nach Anspruch 5, **dadurch gekennzeichnet, dass** diese einen b/l d50-Wert aufweist, für den gilt:

     • Dispergierdruck 5 kPa      : 0,52 bis 1,0 und/oder
     • Dispergierdruck 20 kPa     : 0,55 bis 1,0 und/oder
     • Dispergierdruck 460 kPa   : 0,65 bis 1,0.

7. Verfahren zur Herstellung von kristalliner Allulose gemäß mindestens einem der vorstehenden Ansprüche 1 bis 6, umfassend die folgenden Schritte:

    (a) Bereitstellen einer wässrigen Allulose-Zubereitung;
    (b) Aufkonzentrieren der Zubereitung aus Schritt (a) bis die Sättigungskonzentration überschritten wird;
    (c) Versetzen der übersättigten Zubereitung aus Schritt (c) mit Allulose-Impfkristallen; und
    (d) Abkühlen der Zubereitung aus Schritt (c) mit einer Geschwindigkeit von 0,1 bis etwa 1 K/h bis ein Kristallgehalt von etwa 30 bis etwa 50 Gew.-% erreicht wird, sowie
    (e) Abtrennen und gegebenenfalls Trocknen der Kristalle.

8. Verfahren nach Anspruch 7, **dadurch gekennzeichnet, dass** man wässrige Allulose-Zubereitungen einsetzt, die einen Trockensubstanzgehalt von etwa 55 bis etwa 90 Gew.-% aufweisen.

9. Verfahren nach Anspruch 8, **dadurch gekennzeichnet, dass** man wässrige Allulose-Zubereitungen einsetzt, die einen Trockensubstanzgehalt von etwa 75 bis etwa 85 Gew.-% aufweisen.

10. Verfahren nach mindestens einem der Ansprüche 7 bis 9, **dadurch gekennzeichnet, dass** man der übersättigten Zubereitung Impfkristalle in Mengen von etwa 1 bis etwa 3 Gew.-% - bezogen auf die Zubereitung - zusetzt.

11. Verwendung von kristalliner Allulose nach mindestens einem der Ansprüche 1 bis 6 zur Herstellung von oralen Zubereitungen.

12. Verwendung nach Anspruch 11, **dadurch gekennzeichnet, dass** die oralen Zubereitungen ausgewählt sind aus der Gruppe, die gebildet wird von Nahrungsmitteln, Mund- und Zahnpflegemitteln sowie pharmazeutischen Zubereitungen.

13. Verwendung nach den Ansprüchen 11 und/oder 12, **dadurch gekennzeichnet, dass** die oralen Zubereitungen die kristalline Allulose in Mengen von 0,1 bis etwa 50 Gew.-% enthalten.

Europäisches
Patentamt

European
Patent Office

Office européen
des brevets

## EUROPÄISCHER RECHERCHENBERICHT

**Nummer der Anmeldung**

EP 20 15 6862

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2018/087261 A1 (PFEIFER & LANGEN GMBH & CO KG [DE]) 17. Mai 2018 (2018-05-17) | 1-10 | INV.<br>C07H3/02 |
| Y | * Seiten 28-29, Absatz [0186]-[0190] * <br> * Seite 31, Absatz [0204] * <br> * Seiten 39-40; Beispiele 19-21 * <br> ----- | 11-13 | C07H1/06<br>C07H1/00 |
| X | KR 2019 0003307 A (SAMYANG CORP [KR]) 9. Januar 2019 (2019-01-09) <br> * Zusammenfassung * <br> & EP 3 647 317 A1 (SAMYANG CORP [KR]) 6. Mai 2020 (2020-05-06) <br> * Absätze [[0013]], [[0026]] * <br> * Absätze [[0015]] - [[0016]] * <br> * Absätze [[0031]] - [[0032]] * <br> ----- | 1-13 | |
| Y | EP 2 552 241 A2 (CJ CHEILJEDANG CORP [KR]) 6. Februar 2013 (2013-02-06) <br> * Spalte 5, Absatz [0031] * <br> * Spalte 10; Beispiel 7 * <br> ----- | 1-13 | |
| X | WO 2017/029244 A1 (PFEIFER & LANGEN GMBH & CO KG [DE]) 23. Februar 2017 (2017-02-23) | 1-6 | **RECHERCHIERTE SACHGEBIETE (IPC)** |
| Y | * Ansprüche 1-73 * <br> * Seiten 48-50; Beispiele 1, 2 * <br> ----- | 1-13 | C07H |

Der vorliegende Recherchenbericht wurde für alle Patentansprüche erstellt

| Recherchenort | Abschlußdatum der Recherche | Prüfer |
|---|---|---|
| München | 19. August 2020 | Gohlke, Pascale |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 15 6862

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-08-2020

| Im Recherchenbericht angeführtes Patentdokument | Datum der Veröffentlichung | Mitglied(er) der Patentfamilie | Datum der Veröffentlichung |
|---|---|---|---|
| WO 2018087261 A1 | 17-05-2018 | EP 3538239 A1 | 18-09-2019 |
| | | JP 2019534021 A | 28-11-2019 |
| | | US 2019315790 A1 | 17-10-2019 |
| | | WO 2018087261 A1 | 17-05-2018 |
| KR 20190003307 A | 09-01-2019 | CN 110869379 A | 06-03-2020 |
| | | EP 3647317 A1 | 06-05-2020 |
| | | JP 2020523377 A | 06-08-2020 |
| | | KR 20190003307 A | 09-01-2019 |
| | | TW 201904446 A | 01-02-2019 |
| | | US 2020196648 A1 | 25-06-2020 |
| EP 2552241 A2 | 06-02-2013 | AU 2011230109 A1 | 02-08-2012 |
| | | CN 102250157 A | 23-11-2011 |
| | | EP 2552241 A2 | 06-02-2013 |
| | | JP 2011206054 A | 20-10-2011 |
| | | KR 20110108185 A | 05-10-2011 |
| | | TW 201137129 A | 01-11-2011 |
| | | US 2011237790 A1 | 29-09-2011 |
| | | WO 2011119004 A2 | 29-09-2011 |
| WO 2017029244 A1 | 23-02-2017 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2018087261 A1
- WO 2016135358 A1
- WO 201820103 A1
- WO 2014186084 A1
- WO 20180187261 A1

**In der Beschreibung aufgeführte Nicht-Patentliteratur**

- **H. ITOH et al.** *Journal of Fermentation Bioengeneering,* 1995, vol. 80 (1), 101-103
- **N. WAGNER et al.** *Organic Process Research Development,* 2012, vol. 16, 323-330
- **N. WAGNER et al.** *Chemical Engineering Science,* 2015, vol. 137, 423-435
- **N. WAGNER et al.** *Angewandte Chemie Int. Ed. Engl.,* 2015, vol. 54 (14), 4182-6
- **BOSSHART et al.** *Biotechnology Bioengineering,* 2016, vol. 113 (2), 349-58
- **N. WAGNER et al.** *Journal of Chromatography A,* 2015, vol. 1398, 47-56